# NEW EUROPEAN PATENT SPECIFICATION

(11) **EP 1 635 906 B2**
(45) Date of publication and mention of the opposition decision: **20.02.2019**
(45) Mention of the grant of the patent: 28.03.2012
(21) Application number: 04755009.0
(22) Date of filing: 10.06.2004
(51) Int. Cl.: A61N 1/36

(54) **DEVICE FOR TREATMENT OF DEGENERATIVE RETINAL DISEASE VIA ELECTRICAL STIMULATION OF SURFACE STUCTURES OF THE EYEBALL**
VORRICHTUNG ZUR BEHANDLUNG VON DEGENERATIVEN RETINAERKRANKUNGEN DURCH ELEKTRISCHE STIMULATION VON OBERFLÄCHENSTRUKTUREN DES AUGAPFELS
TRAITEMENT DE MALADIES RETINIENNES PAR STIMULATION DES STRUCTURES SUPERFICIELLES

(30) Priority: 24.06.2003 US 606117; 09.06.2004 US 863519
(43) Date of publication of application: 22.03.2006
(73) Proprietor: Pixium Vision SA, 75012 Paris (FR)
(72) Inventor: CHOW, Alan, Y., Wheaton, IL 60187 (US)
(74) Representative: Graf von Stosch, Andreas
(86) International application number: PCT/US2004/018606
(87) International publication number: WO 2005/004985

(56) References cited:
- EP-A- 0 325 201
- EP-A1- 0 723 984
- EP-A2- 0 325 201
- WO-A1-01/83026
- WO-A1-99/45870
- WO-A2-03/002070
- US-A- 4 955 378
- US-A- 5 109 846
- US-A1- 2003 014 089
- US-A1- 2004 106 965
- US-A1- 2005 004 625
- US-B1- 6 427 087
- CHOW A.Y. ET AL: 'THE ARTIFICIAL SILICON RETINA MICROCHIP FOR THE TREATMENT OF VISION LOSS FROM RETINITIS PIGMENTOSA.' ARCH OPHTHALMOL. vol. 122, no. 4, April 2004, pages 460 - 469
- DECISION OF THE TECHNICAL BOARD OF APPEAL REGARDING EUROPEAN APPLICATION 02780956.5

## Description

### FIELD OF THE INVENTION

The present invention relates to treatment of degenerative retinal disease and, in particular, to apparatus for treatment thereof based on external electrical stimulation.

### BACKGROUND

Many human retinal diseases cause vision loss by partial to complete destruction of the vascular layers of the eye that include the choroid and choriocapillaris, both of which nourish the outer anatomical retina and a portion of the inner anatomical retina of the eye.

Many other retinal diseases cause vision loss due to partial or to complete degeneration of one or both of the two anatomical retinal layers directly, due to inherent abnormalities of these layers. The components of the retinal layers include Bruch's membrane and retinal pigment epithelium which comprise the "outer anatomical retinal layer", and the photoreceptor outer and inner segments, outer nuclear, outer plexiform, inner nuclear, inner plexiform, amacrine cell, ganglion cell and nerve fiber layers which comprise the "inner anatomical retinal layer", also known as the "neuroretina". The outer portion of the neuroretina is comprised of the photoreceptor outer and inner segments and outer nuclear layer (cell bodies of the photoreceptors) and is also known as the "outer retina" which is to be distinguished from the "outer anatomical retinal layer" as defined above. Loss of function of the outer retina is commonly the result of dysfunction of the outer anatomical retinal layer that provides nourishment to the outer retina and/or to direct defects of the outer retina itself. The final common result, however, is dysfunction of the outer retina that contains the light sensing cells, the photoreceptors. Some of these "outer retina" diseases include age-related macular degeneration, retinitis pigmentosa, choroidal disease, long-term retinal detachment, diabetic retinopathies, Stargardt's disease, choroideremia, Best's disease, and rupture of the choroid. The inner portion of the neuroretina, however, often remains functionally and anatomically quite intact and may be activated by the appropriate stimuli.

While researchers have reported efforts to restore visual function in humans by transplanting a variety of retinal cells and retinal layers from donors to the subretinal space of recipients, no sustained visual improvement in such recipients has been widely accepted by the medical community.

Multiple methods and devices to produce prosthetic artificial vision based on patterned electrical stimulation of the neuroretina in contact with, or in close proximity to, the source of electrical stimulation are known. These devices typically employ arrays of stimulating electrodes powered by photodiodes or microphotodiodes disposed on the epiretinal side (the surface of the retina facing the vitreous cavity) or the subretinal side (the underneath side) of the neuroretina. For example, Chow et al. have described various designs for implants to be inserted in the sub-retinal space, i.e., a space created between the inner and outer retinal layers, in U.S. Patent Nos. 5,016,633; 5,024,223; 5,397,350; 5,556,423; 5,895,415; 6,230,057; 6,389,317 and 6,427,087. Generally, the implants described in these patents are placed in contact with the photoreceptor layer of the inner retina such that electrodes on the implants can provide stimulating currents, derived from the photovoltaic conversion of incident light, to the inner retina. Additionally, techniques and devices for inserting such implants into the sub-retinal space are also described in various ones of these patents, e.g., U.S. Pat. Nos. 5,016,633; 5,024,223 and 6,389,317.

US 2003/0014089 A1 describes methods for improving damaged cell functions, wherein a retina stimulation device including a plurality of electrodes is disposed in the subretinal space of the eye or is implanted between the conjunctiva and a scleral surface.

WO 99/45870 A1 describes a visual prosthesis including an electrode array and a decoding and demultiplexing circuitry, wherein the electrode array is disposed on the retina, and the circuitry is disposed on an outer surface of the sclera.

WO 01/83026 A1 describes an artificial retina device with stimulating and ground return electrodes disposed on opposite sides of the neuroretina, wherein a ground return electrode may be disposed on a scleral surface on an outside of an eye.

Cellular electrical signals also play important developmental roles, enabling nerve cells to develop and function properly. For example, nerve cells undergo constant remodeling, or "arborization", during development related to electric signaling. First an extensive preliminary network is formed that is then "pruned" and refined by mechanisms that include cell death, selective growth, loss of neurites (axonal and dendritic outgrowths), and the stabilization and elimination of synapses (Neely and Nicholls, 1995). If a neuron fails to exhibit or is inhibited from transducing normal electrical activity during arborization, axons fail to retract branches that had grown to inappropriate positions.

The application of electric currents to organ systems other than the eye is known to promote and maintain certain cellular functions, including bone growth, spinal cord growth and cochlear spiral ganglion cell preservation (Acheson et al., 1991; Dooley et al., 1978; Evans et al., 2001; Kane, 1988; Koyama et al., 1997; Lagey et al., 1986; Leake et al., 1991; Leake et al., 1999; Politis and Zanakis, 1988a; Politis and Zanakis, 1988b; Politis and Zanakis, 1989; Politis et al., 1988a; Politis et al., 1988b).

In other studies, the application of growth and neurotrophic-type factors was found to promote and maintain certain retinal cellular functions. For example, brain-derived neurotrophic factor (BDNF), neurotrophin-4 (NT-4), neurotrophin-5 (NT-5), fibroblastic growth factor (FGF) and glial cell line-derived neurotrophic factor (GDNF) have been shown to enhanced neurite outgrowth of retinal ganglion cells and to increase their survival in cell culture. GDNF has been shown to preserve rod photoreceptors in the rd/rd mouse, an animal model of retinal degeneration. Nerve growth factor (NGF) injected into the intra-ocular area of the C3H mouse, also a model of retinal degeneration, results in a significant increase of surviving photoreceptor cells compared to controls (Bosco and Linden, 1999; Caleo et al., 1999; Carmignoto et al., 1989; Cui et al., 1998; Frasson et al., 1999; Lambiase and Aloe, 1996; Reh et al., 1996). No methods or devices, however, to improve the general inherent visual function of damaged retinal cells distant from a source of electrical stimulation through the use of chronic electrical stimulation applied to the neuroretina from either within the eye or indirectly via contact with surface structures of the eye are known.

Further examples of known devices for the treatment of degenerative retinal disease are described in US Patent No. 5,109,846 A and US Patent Application Publication No. 2003/014089 A1. Other similar devices are described in European Patent Application Publication No. EP 1 723 984 and International Patent Application Publication No. WO 2004/067088, both published after the present application.

### BRIEF SUMMARY OF THE INVENTION

The present invention provides devices for preventive or therapeutic treatment of degenerative retinal disease through the application of electrical stimulation. In particular, the present invention concerns the use of electrical stimulation applied to surface structures of an eyeball for such treatment, the surface structures being arranged posterior to the bulbar conjunctiva and comprising the various laminae beginning with the sclera as the innermost surface structure and its overlying structures. Generally, this is achieved with a device comprising a source of an electrical stimulation signal coupled to a first electrode configured for contact with a first internal surface structure of an eyeball and a second electrode configured for contact with a second surface structure of the eyeball, which second surface structure may be external or internal. Surface structures of the eyeball may be categorized as either external surface structures (e. g., conjunctiva and cornea) or internal surface structures (e. g., sclera, episclera, intramuscular septum, Tenon's capsule, extraocular muscles or tendon, etc.). The source of the electrical stimulation signal may be implemented internal to a body of a patient, external to the body or through a combined internal/external approach. The source comprises an internal source component exhibiting a receiver induction coil implanted subcutaneously and further comprises an external source component exhibiting a transmitting coil configured for transmitting power and data. Electrodes in accordance with various embodiments of the present invention may be arranged in one or more ring formations, including interleaved electrodes. The electrodes are preferably arranged such that a circuit created by the source, electrodes and intervening biological tissue provides trans-retinal electrical stimulation to thereby effect treatment.

### BRIEF DESCRIPTION OF THE DRAWINGS

FIG. 1 is a cross-sectional top view of a human eye.
FIG. 2 is a cross-section through a human eye indicating layers of the outer and inner anatomical retina, as indicated by the inset of FIG. 1.
FIG. 3 is a schematic block diagram of a prior art technique for indirect electrical stimulation.
FIG. 4 is a schematic block diagram of another prior art technique for indirect electrical stimulation.
FIG. 5 is a schematic block diagram of yet another prior art technique for indirect electrical stimulation.
FIG. 6 is a schematic block diagram of a technique for indirect electrical stimulation in accordance with the present invention.
FIG. 7 is a schematic block diagram of another technique for indirect electrical stimulation in accordance with the present invention.
FIG. 8 is a partial cross-sectional side view of a human eye and surrounding structures.
FIG. 9 is a partial cross-sectional magnified view of a region of the eye illustrated in FIG. 8.
FIG. 10 is a side view of a human eye illustrating application of a corneal electrode in accordance with an embodiment of the present invention.
FIG. 11 is a side view of a human eye illustrating application of an epi-conjunctival electrode in accordance with an embodiment of the present invention.
FIG. 12 is a side view of a human eye illustrating application of a fiber electrode in a conjunctival fornix in accordance with an embodiment of the present invention.
FIG. 13 is a side view of a human eye illustrating application of a plurality of electrode arrays applied to an internal surface structure in accordance with an embodiment of the present invention.
FIG.14 is a side view of a human eye illustrating application of an electrode array to an internal surface structure in accordance with an embodiment of the present invention.
FIG. 15 is a schematic block (diagram of an internal surface structure/internal surface structure technique for indirect electrical stimulation in accordance with the present invention.
FIG. 16 is a schematic block diagram of an internal surface structure/external surface structure technique for indirect electrical stimulation in accordance with the present invention.
FIG. 17 is a top view of a human eye illustrating an exemplary implementation of the embodiment of FIG. 15.
FIG. 18 is a top view of a human eye illustrating a first
   exemplary implementation of the embodiment of FIG. 16 which is not part of the invention.
FIG. 19 is a top view of a human eye illustrating a second implementation of the embodiment of FIG. 16 which is not part of the invention.

### DETAILED DESCRIPTION OF THE PRESENTLY PREFERRED EMBODIMENTS

In the course of testing for the safety and efficacy of retinal implants in humans blinded by retinitis pigmentosa, an unexpected and surprising observation was made: even though the implants were placed at a discrete location in the subretinal space (acting as a prosthesis), vision was improved not only in those discrete locations as expected, but also in distant locations of the retina. Thus chronic electrical stimulation in specific locations enhanced retinal cell function throughout the eye. This "halo effect" can be used to improve vision in those individuals who suffer from diseases, conditions and traumas that have damaged the outer retinal layer but leave the inner retinal layer at least partially intact. Although prosthetic electrical devices designed to replace damaged or missing retinal cells have been used to treat vision loss caused by outer retinal degeneration, electrical stimulation to improve large areas of retinal cell visual function is novel. As a non-limiting explanation, the promotion of improved retinal cell visual function by chronic electrical stimulation may be explained by the stimulation of production and release of growth factors; more specifically, neurotrophic-type growth factors, by the stimulated retinas. The synthesis and/or secretion of neurotrophic factors would then improve retinal cell function and survival in conditions where these activities would be lost.

Accordingly, the present invention discloses novel devices to electrically stimulate the retina to improve large areas of retinal visual function and to protect the retina from degeneration. As described in greater detail below, the devices disclosed herein may be generally categorized as indirect. Direct techniques involve stimulation of a retina wherein the stimulus traverses substantially no intervening biological structures. Conversely, indirect techniques encompass stimulation of a retina wherein the stimulus must traverse one or more intervening biological structures.

### Subject/Patient

A subject (patient) may be a human being or a non-human animal, but is preferably a human. Usually the individual has suffered some type of retinal damage and/or degeneration that results in some degree of visual loss and/or has a condition that will result in retinal damage and/or degeneration. A normal (healthy) subject does not have a condition that will result in retinal damage and/or degeneration and/or has not suffered retinal damage and/or degeneration.

### Improving visual function

Improving visual function refers to improving a targeted function of the eye, selected by the artisan, and includes improving any to all of the following capabilities of the eye, retina and visual system: perception of brightness in the presence of light, perception of darkness in the absence of light, perceptions of contrast, color, shape, resolution, movement and visual field size.

Primary visual degradation means loss of visual function due to malfunctioning, damaged or degeneration of structures found in the eye. Secondary visual degradation means loss of visual function due to secondary damage, typically from lack of use of the vision-associated portions of the brain. Improving visual function means to improve the visual function of primary visual degradation, secondary visual degradation or both.

### Eye/eyeball

The eye (or eyeball) has the usual definition in the art. Eye includes all interior and exterior surfaces, components, contents and cavities of the eye. The eye does not include the eyelid or optic nerve.

The retina of the eye can be divided into sectors as is commonly accepted in the art. Such sectors are described by the use of the terms temporal, nasal, superior, inferior, by clock hour designation, and by the number of degrees away from the macula. For example, the temporal sector of the retina is the retina temporal to a perpendicular plane cutting through retina from the 12 o'clock to the 6 o'clock positions and through the macula. In another example, the superior sector is the retina superior to a perpendicular plane cutting through the 9 o'clock to 3 o'clock positions and through the macula. In a further example, the superior-temporal sector is the intersection of these two sectors, a pie-shaped area delineated from the 9 o'clock position of the peripheral retina to the macula and then clockwise to the 12 o'clock position. More specific locations of the retina can be designated by degrees away from the macula and clock hour location: for example, 20 degrees away from the macula at the 3 o'clock (nasal) position. The number of degrees away from the macula is in visual axes degrees. These axes all intersect through the lens of the eye.

The visual field sectors correspond oppositely to the retinal sectors as is commonly understood in the art. For example, the superior-temporal sector of the retina corresponds to the inferior-nasal portion of the visual field.

### Peripheral

To be peripheral to an object, device or other landmark includes all surrounding parts, but not the object, device or landmark, *i.e.,* the object, device or landmark, together with the peripheral portion, constitutes the whole.

### Light

Light refers not only to the electromagnetic spectrum that humans can readily perceive visually (approximately 400 nm to 750 nm), but also includes ultraviolet light (<400 nm in wavelength) as well as infrared light (>750 nm in wavelength).

### Indications

The invention can be used to improve visual function in subjects in which the retina is damaged by disease, degeneration, condition, or trauma and/or to slow down or stop the progression of damage by disease, degeneration, condition or trauma. Common diseases, conditions, degeneration or trauma that are particularly amenable to this treatment include age-related macula degeneration, retinitis pigmentosa, Leber's congenital amaurosis, Stargardt's disease, Best's disease, diabetic retinopathy, long-term retinal detachment, and choroidal damage.

### Eye structure

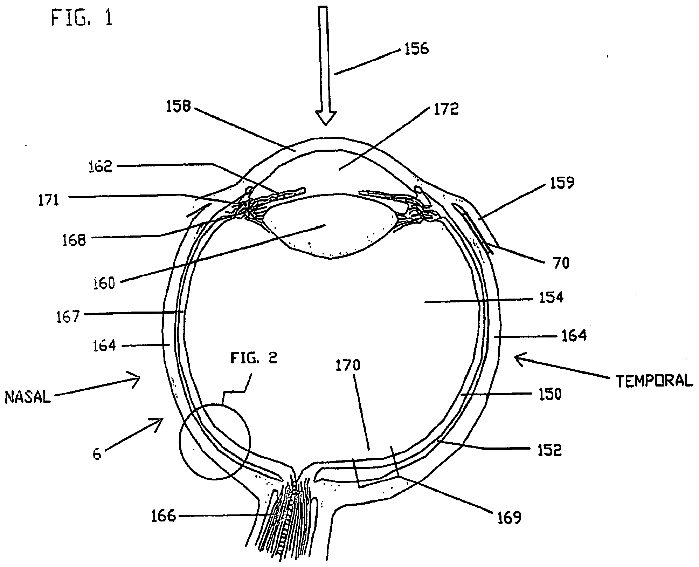

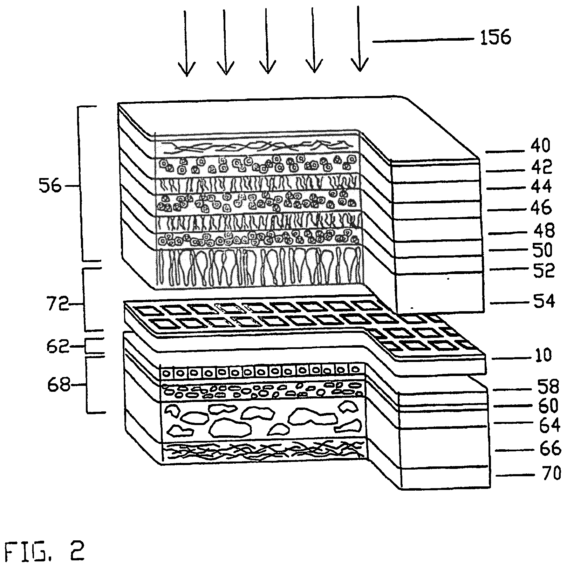

Referring to the drawings, FIG. 1 illustrates a section through the eyeball. The neuroretina 150 comprises multiple layers of cells and structures (see FIG. 2). The photoreceptor components of the retina are situated within the neuroretina which covers the internal posterior cavity of the eye, terminating anteriorly at the ora serrata 167. The ciliary body 168 and the iris 162 are covered by extensions of the retina, lacking photoreceptor components. The outermost layers of the eye consist of the sclera 164 and cornea 158. The sclera is pierced by the emerging optic nerve 166. The lens 160 and vitreous cavity 154 are also indicated. The macula 169 of the retina is typically a 3 mm by 5 mm oval region, at the center of which is the fovea 170.

The layers of the eye at the posterior pole from inside to outside are shown in FIG. 2: internal limiting membrane 40, nerve fiber layer 42, ganglion cell layer 44, inner plexiform 46, inner nuclear layer 48, outer plexiform 50, outer nuclear cell layer 52, and photoreceptor outer and inner segment layer 54, all of which constitute the anatomical inner retinal layer, also known as the neuroretina 56. The retinal pigment epithelium 58 and Bruch's membrane 60 constitute the outer retinal layer 62. The choriocapillaris 64 and choroid 66 comprise the choroidal vasculature 68. The outer coat of the eye is the sclera 70. Light 156 enters the retina as shown.

### Indirect Stimulation

In prior applications, embodiments were disclosed processing a common characteristic that the electrical stimulus is provided directly to the neuroretina, i.e., there are substantially no intervening biological structures. In accordance with the present invention, electrical stimulus may be applied to the neuroretina in an indirect fashion, i.e., via one or more intervening biological structures.

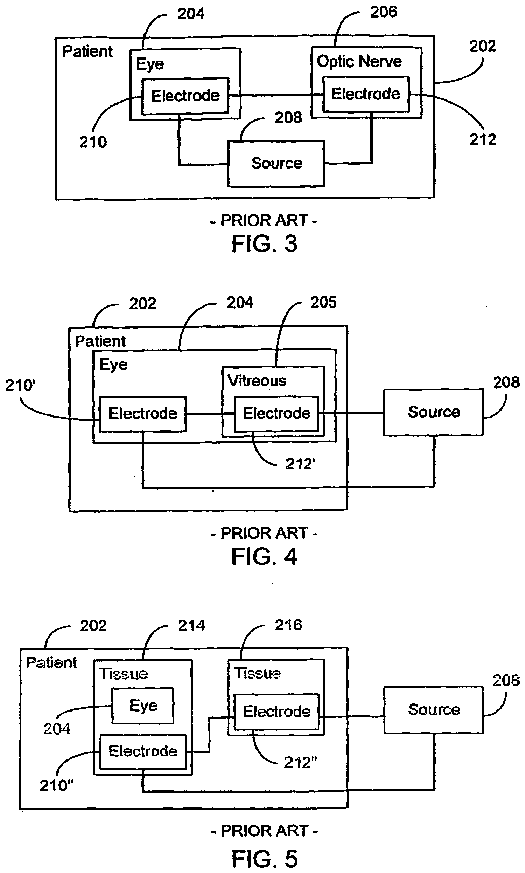

Various methods for indirect stimulation, as that term is defined herein, are known. FIGs. 3-5 are schematic illustrations of such prior art techniques. FIG. 3 illustrates a technique (described in U.S. Patent No. 5,147,284 issued to Fedorov et al.; hereinafter "Fedorov") in which electrical stimulation is applied to an eye 204 of a patient 202 via a pair of surgically implanted electrodes 210, 212 applied to surfaces of the eye 204 and optic nerve 206. A source of electrical stimulation 208 is provided coupled to the pair of electrodes 210, 212. In practice, the source 208 comprises an induction coil that provides electrical currents as a result of magnetic fields applied to the temporal region of the patient 202. While Fedorov reports improved vision in patients, the circumstances under which the patients were treated are not known and do not appear to have been subjected to peer review. Moreover, it will be readily evident to those having ordinary skill in the art that the implantation of an electrode in close proximity to the optic nerve 206 requires highly invasive and complicated surgery.

FIG. 4 illustrates a more recent technique proposed by Chow in U.S. Patent No. 6,427,087. In particular, an electrode 210' is placed in contact with tissues of the eye 204, whereas another electrode 212' is placed within the vitreous cavity 205 that may be in contact with the internal limiting membrane (see also the vitreous cavity 154 illustrated in FIG. 1). It is believed that the resulting trans-retinal stimulation resulting from this configuration will result in more efficient stimulation.

Yet another approach is illustrated in FIG. 5 in which the stimulating 210" and return 212" electrodes, rather than being in direct contact with the eye 204, are instead placed upon external tissues 214, 216. Examples of this approach (sometimes referred to as microcurrent stimulation), particularly for the purpose of treating degenerative retinal diseases such as macular degeneration and retinitis pigmentosa, are taught in U.S. Patent No. 5,522,864 to Wallace et al. and U.S. Patent Nos. 6,035,236 and 6,275,735 to Jarding et al. Typically, the stimulating electrode 210" is coupled to external tissue in close proximity to the eye 204, e.g., the eyelid, and the return electrode 212" is coupled to distal external tissues such as the occipital lobe or arm of the patient 202. While anecdotal evidence of efficacy has been sporadically reported, no controlled, peer reviewed studies on humans are known to have been performed and, furthermore, the American Academy of Ophthalmology's Task Force on Complementary Therapies concluded in September 2000 that "strong evidence has not been found to demonstrate the effectiveness of microcurrent stimulation treatment of [age-related macular degeneration] compared to standard therapies."

In contrast to the prior art techniques described above, the present invention encompasses indirect stimulation techniques based on application of one or more electrodes to surface structures of the eye, as opposed to peripheral structures such as the optic nerve or eyelids. As used herein, surface structures of the eye may be divided into two classes, internal surface structures and external surface structures as described in greater detail below. In general, surface structures of the eye may be defined as any of several laminae (beginning most interiorly with the sclera in the case of internal surface structures) and forming or surrounding the eye, depending upon the specific region of the eye under consideration.

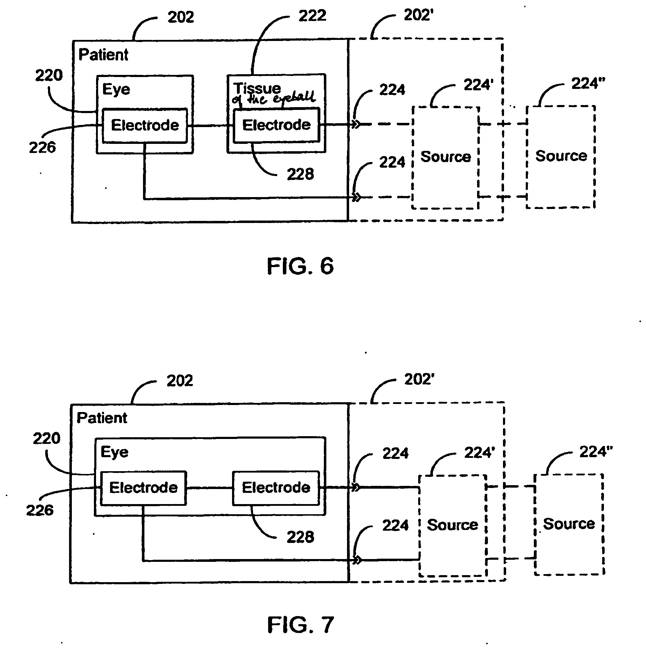

A schematic illustration of an embodiment of indirect stimulation in accordance with the present invention is presented in FIG. 6. In this embodiment, at least one active or stimulating electrode 226 is applied to a surface structure of an eye 220. The at least one active electrode 226 is configured for chronic contact with the surface structure of the eye 220. As used herein, the term chronic encompasses not only continuous periods of time but also repetitive and/or periodic intervals of time. For example, the at least one active electrode 226 may be substantially permanently attached or otherwise coupled to the surface structure, or it may be configured to allow for repetitive placement in contact with, and subsequent removal from, the surface structure over a period of time established by a course of treatment. At least one return or ground electrode 228 is configured for application to tissues 222 of an external surface structure of the eyeball or to an internal surface structure of the eyeball being an outer surface of the sclera of the patient. Additionally, the at least one return electrode 228 may be configured for chronic or temporary application to the tissue 222. For example, the at least one return electrode 228 may comprise one or more implantable electrodes substantially permanently coupled to the tissue 222 or it may comprise one or more temporary cutaneous electrodes secured with an adhesive and electrically coupled using a suitable conductive gel. Positioned in this manner, and given the relatively low resistance of the vitreous relative to the surface structures and surrounding tissues of the eye, the active and ground return electrodes establish a trans-retinal circuit such that application of an electrical stimulation signal to the active electrode will result in beneficial trans-retinal currents.

In addition to the electrodes 226, 228, the above system illustrated in FIG.6 also comprises a source of the electrical stimulation signal. The particular configuration of the source depends on internal and external implementation, relative to the patient. For example, in the case where only the active electrode 226 is configured to removably contact external surface structures of the eye and the return electrode 228 is configured for temporary cutaneous contact, the source may comprise one or more input terminals 224 for application of the electrical stimulation signal to the electrodes. In this case, the electrical stimulation signal is provided by an extraocular signal source 224'.

According to a non-inventive embodiment which does not form part of the invention, the source 224' is entirely internal to the patient 202' as in the case of an implantable battery and, optionally, signal generation circuitry (not shown). In this case, it is assumed that the at least one return electrode 228 is likewise chronically implanted in the patient 202', thereby vitiating the need for any input terminals 224.

The source is implemented as a combination of internal 224' and external 224" (relative to the patient) components. The internal source component 224' exhibits a receiver induction coil implanted subcutaneously and the external source component 224" exhibits a transmitting coil that may be precisely aligned with the receiver induction coil. As known in the art, such transmitter/receiver coil pairs may be used to transmit power and data that may be used to provide the electrical stimulation signal.

In practice, the electrical stimulation signal provided by the source may comprise virtually any type of waveform demonstrating a beneficial effect. For example, the electrical stimulation signal may comprise an anodic or cathodic direct current signal or a time-varying waveform such as a square, sine, triangular, saw tooth signal or any other similar waveform. Preferably, the electrical stimulation signal comprises a bi-phasic waveform that is balanced in the sense a net zero charge is applied to the retina over a period of time. This may be achieved, by way of non-exhaustive examples, through the use of a signal comprising a continuous train of equal-duration bi-phasic pulses; equal-duration bi-phasic pulses separated by periods of quiescence; varying duration and amplitude bi-phasic, charge balanced pulses; combinations of the above; etc. Pulse frequencies may range anywhere from 10KHz down to 0.001 Hz or, in the extreme, even a continuous monophasic waveform, i.e., 0 Hz. Those having ordinary skill in the art will appreciate that the particular type of electrical stimulation signal used is a matter of design choice and is selected so as to provide maximum beneficial effect.

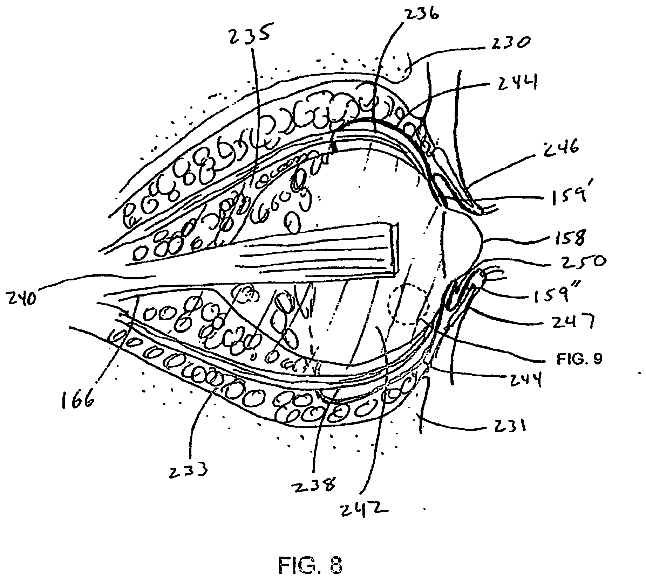

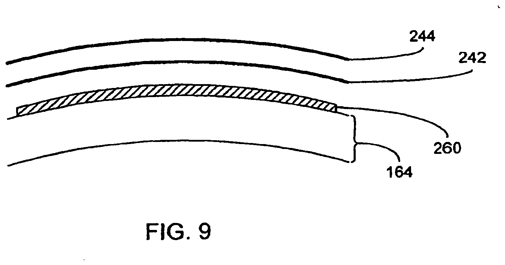

A schematic illustration of another embodiment of indirect stimulation in accordance with the present invention is presented in FIG. 7. In this embodiment, the at least one active electrode 226 is applied to a first surface structure of the eye 220 and the at least one return electrode 228 is applied to a second surface structure of the eye 220. In practice, the first and second surface structures may be the same or different surface structures, the surface structures comprising the various laminae beginning with the sclera as the innermost surface structure and its overlying structures. The source 224, 224', 224" of the electrical stimulation signal in this embodiment may comprise any of the alternatives described above relative to FIG. 6. It is anticipated that the embodiment of indirect stimulation illustrated in FIG. 7 will provide heightened stimulation of the retina given the relative proximity of electrodes to the retina. The various surface structures applicable to the present invention are further described below with reference to FIGs. 8 and 9.

Referring now to FIG. 8, an eye and surrounding structures are illustrated. The ocular orbit is defined by bone structures 230, 231. Within the orbit, a layer of extraconal fat 233 and intraconal fat 235 surround the eyeball. The fat layers 233, 235 are separated from each other by a cone defined by superior 236, inferior 238 and lateral 240 extraocular muscles as well as an intermuscular septum 242 connecting the muscles. The optic nerve 166 exits the orbit posteriorly, whereas the anterior portion of the eyeball is formed by a portion of the sclera and the cornea 158. The so-called Tenon's capsule 244 (partially shown) separates the eyeball from the orbital fat and forms a socket within which the eyeball moves. The upper and lower eyelids 246, 247 enclose and protect the anterior portion of the eyeball. The conjunctiva comprises the bulbar conjunctiva 159' overlying the anterior portion of the sclera and the palpebral conjunctiva 159" overlying the inner surface of the upper and lower eyelids 246, 247. The fold between the bulbar and palpebral conjunctiva 159', 159" gives rise to a conjunctival fornix 250. In the context of the present invention, external surface structures comprise those surface structures that are accessible via the palpebral fissure defined by the eyelids, i.e., the cornea 158 and the conjunctiva 159. Internal surface structures are defined as those surface structures posterior to the bulbar conjunctiva 159' and comprise the various laminae beginning with the sclera and its overlying structures, which overlying structures are dependent upon the particular region of the eyeball under consideration.

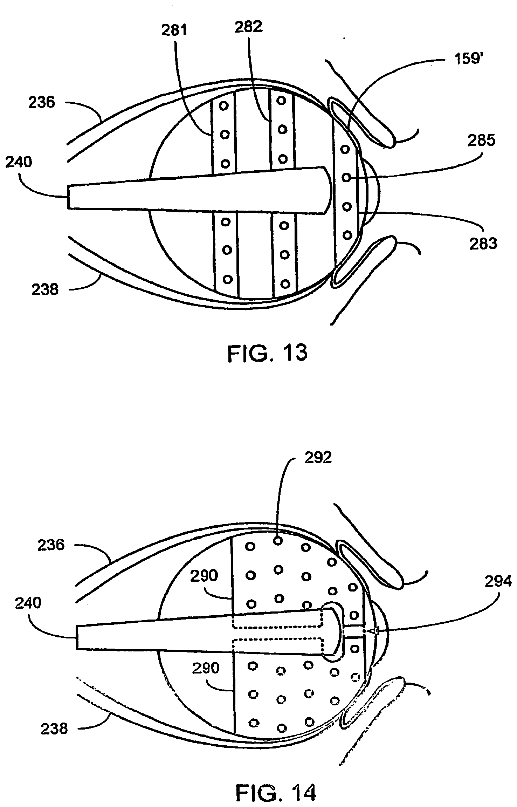

FIG. 9 schematically illustrates the various surface structures present at the exemplary region indicated in FIG. 8. The dimensions shown are not to scale. The sclera 164 forms the innermost surface structure. Moving outwardly from the sclera 164, the episclera 260 is a thin, loose layer of connective tissue forming the outer surface of the sclera 164. The intermuscular septum 242 resides above the episclera 260, and Tenon's capsule 244 resides above the intermuscular septum 242. Each of the layers illustrated in FIG. 14 comprises, for purposes of the instant invention, a separate surface structure to which an electrode may be applied. Those having ordinary skill in the art will appreciate that other regions of the eyeball may have surface structure layers different from those illustrated in FIG. 9.

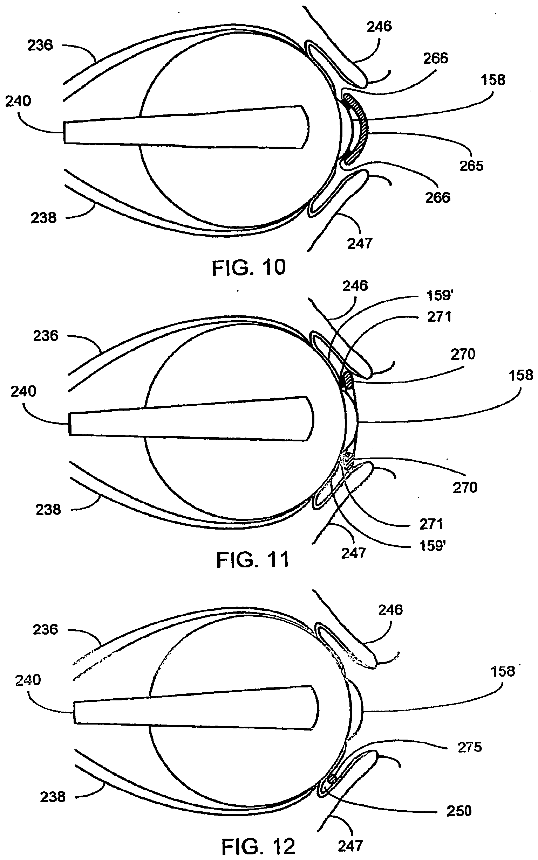

Various exemplary implementations of the embodiments of FIGs. 6 and 7 are schematically illustrated with respect to FIGs. 10-14. For reference, each of FIGs. 10-12 illustrate the superior 236, inferior 238 and lateral 240 extraocular muscles, the upper and lower eyelids 246, 247 and the cornea 158. FIG. 10 illustrates an embodiment in which a contact lens body 265 supports one or more corneal electrodes 266. Various materials for fabricating the supporting body 265 and the at least one corneal electrode 266 are known to those having ordinary skill in the art. Corneal electrode 266 is employed as a return electrode. (This is also true of the other embodiments illustrated in FIGs. 11 and 12.) The at least one corneal electrode 266 may comprise a plurality of discrete electrodes arranged, for example, in a ring formation affixed in proximity to the periphery of the supporting body 265, or may comprise a single annular electrode similarly affixed to the supporting body 265. Alternatively, the at least one corneal electrode 266 may be arranged closer to the central region of the cornea. Note that, for ease of illustration, none of FIGs. 10-12 illustrate the complementary electrode, nor do FIGs. 10-14 illustrate the electrical connections between the electrodes and the source of the electrical stimulation signal, which connections will be readily devisable as a matter of design choice by those having ordinary skill in the art.

FIG. 11 illustrates another embodiment in which an annular supporting body 270 provides support for at least one epi-conjunctival electrode 271. Once again, various materials for fabricating the supporting body 270 and the at least one epi-conjunctival electrode 271 are known to those having ordinary skill in the art. As in the embodiment of FIG. 10, the at least one epi-conjunctival electrode 271 may comprise a plurality of individually selectable electrodes or a single annular electrode as a matter of design choice. In the example shown in FIG. 11, the at least one epi-conjunctival electrode 271 contacts the bulbar conjunctiva 159' in close proximity to the cornea 158. However, additionally or alternatively, the at least one epi-conjunctival electrode 271 may be placed more distally from the cornea 158 and yet still in contact with the bulbar conjunctiva 159'.

FIG. 12 illustrates yet another embodiment in which an electrode 275 is placed in epi-conjunctival contact within the conjunctival fornix 250. In practice, the electrode 275 may comprise a fibrous or filamentary electrode such as a "DTL" electrode. DTL electrodes are particularly advantageous because they are known to be well tolerated by patients given their relatively slender dimensions. Although a single electrode is illustrated in the lower conjunctival fornix 250, an electrode may also be placed in the upper conjunctival fornix as an alternative, or in addition to, the lower electrode. Furthermore, more than one electrode can be placed into either of the fornices at a single time.

Each of FIGs. 10-12 illustrates embodiments in which second electrodes are placed in contact with external surface structures of the eye. FIG. 13 schematically illustrates an embodiment in which electrodes are applied to internal surface structures. In particular, one or more supporting rings 281-283 are implanted in contact with internal surface structures. Note that although the third ring 283 is placed in contact with a substantially anterior portion of the eye, it is implanted beneath the bulbar conjunctiva 159'. However, a hybrid internal/external surface structure technique may be possible if the third ring 283 were placed above the bulbar conjunctiva 159' in a manner similar to that illustrated in FIG. 11. (Such hybrid arrangements are further described with reference to FIGs, 16, 18 and 19 below.) Techniques for introducing such rings into the orbit and for securing them to the eye are know in the art, particularly from the use of so-called scleral buckles. For example, each ring may be sutured in place in accordance with such techniques. Note that the first and second rings 281, 282 are preferably placed beneath the extraocular muscles 236, 238, 240 in accordance with known techniques.

Each ring comprises at least one electrode 285 and, in a preferred embodiment, each ring comprises a plurality of electrodes. Suitable materials for fabricating the supporting rings and electrodes are known to those having ordinary skill in the art. Preferably, each electrode is individually selectable. Additionally, each individual electrode may be electrically configured to act as an active electrode or a return electrode. In this manner, each ring 281-283 may comprise both active and return electrodes. In such an embodiment, it may be preferable to interleave active and return electrodes and, further, to antipodally arrange the active and return electrodes. An antipodal arrangement of electrodes will give rise to a trans-retinal current path that is substantially perpendicular to the retinal surface, Additionally, being individually selectable, each electrode in an antipodal electrode pair could be periodically switched between active and return operation. Further still, electrodes between rings could be activated as stimulating pairs, e.g., an electrode from a first ring 281 could be operated as an active electrode and an electrode from a second ring 282 could be operated as a return electrode, and vice versa. Although a specific number of supporting rings 281-283 positioned in substantially vertical orientations are illustrated in FIG. 13, it is understood that a greater or lesser number of such rings could be employed and, further, that the orientation of such rings need not be limited to substantially vertical. Taken to an extreme, the supporting rings 281-283 could be eliminated and, instead, each electrode 285 may comprise a separate, independent supporting member such that individual electrodes may be implanted at specific locations on specific internal surface structures.

Yet another embodiment providing contact with internal surface structures is illustrated in FIG. 14. In this embodiment, one or more supporting sheaths 290 comprising a plurality of electrodes 292 are positioned and secured in contact with internal surface structures of the eye. The discussion above with regard to individually selectable and antipodal electrodes relative to FIG. 13 equally applies to the arrangement of FIG. 14. To accommodate the presence of various ocular structures, such as the connections between the various muscles 236, 238, 240 and the sclera, openings 294 may be provided. In the example illustrated in FIG. 14, a plurality of sheaths 290 are provided such that each sheath 290 covers the surfaces between adjacent muscles with the openings 294 thereby being formed by the adjacency of the sheaths 290 when implanted. In the case where a single sheath 290 surrounds at least one of the muscles, the anterior portion of the opening 294 could be fabricated such that a unitary body is provided (illustrated with dotted lines) whereas the opening of the posterior portion of the opening 294 would allow flexing of the sheath 290 for placement underneath the muscle. Further still, rather than trying to maneuver the sheaths 290 around the muscles, holes could be provided within the otherwise continuous sheaths 290. In this case, the muscles would need to be severed first to allow positioning of the sheaths, followed by reattachment of the muscles at positions corresponding to the holes. Regardless of the particular configuration, the embodiment illustrated in FIG. 14 allows multiple electrodes to be placed in contact with internal surface structures of the eye to facilitate indirect stimulation of the retina.

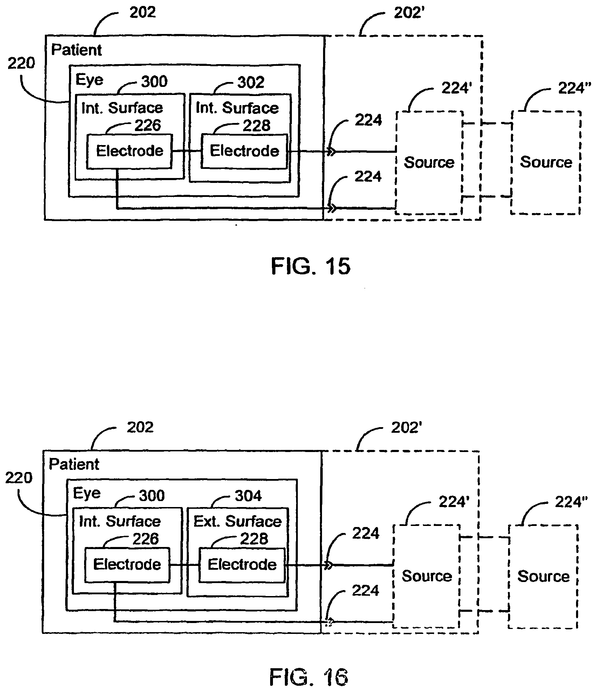

The embodiments of FIGs. 13 and 14 are particular examples of the schema illustrated in FIG. 15. Similar to the schemas illustrated in FIGs. 6 and 7, the schema of FIG. 15 comprises a source 224, 224', 224" coupled to electrodes 226, 228 in contact with an eye 220. However, in this schema, the electrodes are both in contact with internal surface structures 300, 302 of the eye. In a presently preferred embodiment, the electrodes 226, 228, being configured for contact with internal surface structures, are preferably configured for chronic implantation, e.g., constructed of materials exhibiting very good biodurability, biocompatibility, etc.

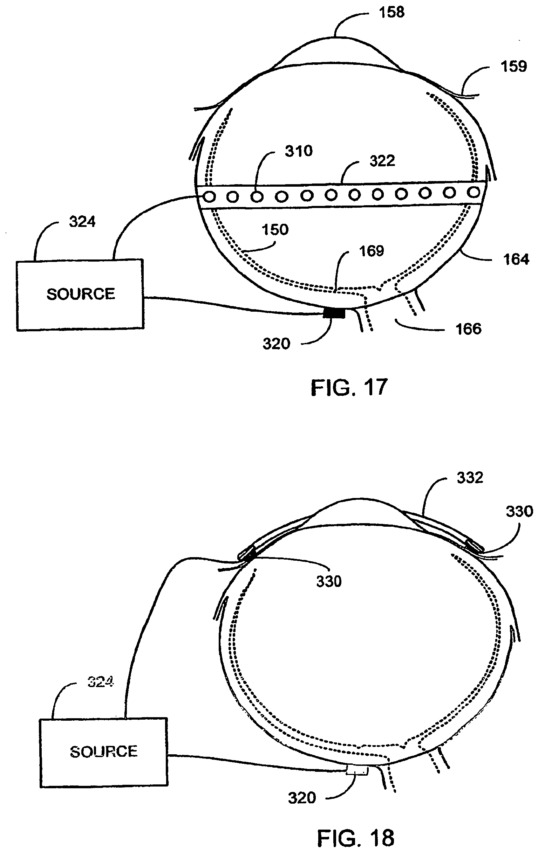

A further schema (generalizing the "hybrid" embodiment mentioned above with regard to FIG. 13) is illustrated in FIG. 16. The schema of FIG. 16 differs from that of FIG. 15 in that one second electrode 228 is configured for contact with an external surface structure 304 while the other first electrode 226 is configured for contact with an internal surface structure 300. Once again, electrodes 226 configured for contact with internal surface structures are preferably configured for chronic implantation. In contrast, those second electrodes 228 configured for contact with external surface structures may be configured for chronic or acute (i.e., removable) contact. For the embodiments illustrated in FIGs. 15 and 16, as in all previous embodiments, the electrodes 226, 228 may each comprise a plurality of separately selectable electrodes each of which may be alternated between stimulating and return electrode functionality. Particular implementations of the schemas of FIGs. 15 and 16 are further illustrated in FIGs. 17-19. For reference, each of FIGs. 17-19 illustrate a top view of an eyeball comprising a cornea 158, conjunctiva 159, sclera 164 and, in hidden view, a neuroretina 150, macula 169 and optic nerve 166.

Referring now to FIG. 17, an exemplary embodiment of the schema of FIG. 15 is illustrated. In particular, FIG. 17 illustrates the placement of a first electrode 310 (in this case, a plurality of electrodes) placed on a first internal surface structure and a second electrode 320 on a second internal surface structure. Connections to an electrical source 324 are schematically shown. In this embodiment, the first and second internal surface structures may comprise, for example, separate regions of scleral tissue. As shown, the plurality of electrodes 310 constituting the first electrode is supported by a body member 322 in the form of a scleral band or ring, as described previously with reference to FIG. 13. The second electrode 320, although illustrated as a single electrode, may comprise a plurality of electrodes. In either case, the second electrode 320 may include a supporting body member (not shown) to aid in positioning and fixation of the second electrode. Applying an electrical stimulation signal across the first and second electrodes will result in trans-retinal currents that stimulate the retina. To maximize the effects of trans-retinal current, the second electrode 320 is preferably configured for and positioned upon an internal surface structure corresponding to (i.e., aligned with) the macula 169 of the eye.

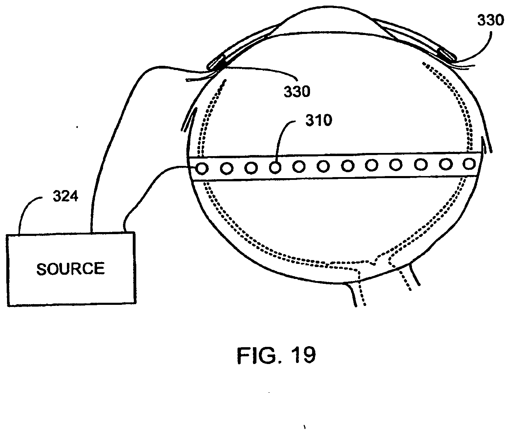

Referring now to FIGs. 18 and 19, embodiments are illustrated, which are not part of the invention. FIG. 18 illustrates placement of a second electrode 320 (as described above relative to FIG. 17) on an internal surface structure of the eye. However, in this embodiment, the first electrode 330 is configured for epi-conjunctival placement, i.e., in contact with an external surface structure. As shown, the first electrode may comprise a supporting body member 332 as described previously with reference to FIG. 11. FIG. 19 illustrates yet another internal/external surface structure embodiment combining a scleral ring electrode 310 implementation and an epi-conjunctival electrode 330 implementation.

## Claims

1. A device for treatment of degenerative retinal disease, comprising:
a source (224, 224', 224") of electrical stimulation;
a first electrode (226), coupled to the source (224, 224', 224"), configured for contact with a first internal surface structure (300) of an eyeball (220) posterior to the bulbar conjunctiva and comprising the various laminae beginning with the sclera as the innermost surface structure and its overlying structures; and
a second electrode (228), coupled to the source (224, 224', 224"), configured for contact with a second surface structure of the eyeball (220);
**characterized in that** the source (224) comprises an internal source component (224') exhibiting a receiver induction coil implanted subcutaneously and further comprises an external source component (224") exhibiting a transmitting coil configured for transmitting power and data, wherein the second electrode (228) is configured for contact with an external surface structure (158, 159, 304) of the eyeball, or **in that** the second electrode (228) is configured for attachment to an internal surface structure of the eyeball being an outer surface of the sclera.

2. The device of claim 1, wherein the second electrode (228) is supported on a body member (281-283, 290) configured for attachment to the outer surface of the sclera.

3. The device of claim 2, wherein the body member (281-283, 290) comprises a band, a ring or a sheath which is configured to extend at least partially around the eyeball to aid in positioning and fixation of the second electrode.

4. The device of claim 3, wherein the body member (281-283, 290) includes one or more openings to accommodate the presence of ocular structures, such as connections between muscles and the sclera.

5. The device of claim 1, wherein the first electrode (226) is at least one stimulating electrode configured for chronic contact with the first surface structure of the eyeball, and the second electrode (228) is at least one return electrode configured for chronic contact with the second surface structure of the eyeball.

6. The device of claim 5, wherein an electrical stimulation signal from the source (224, 224', 224") is applied to the eyeball (220) via the at least one stimulating electrode and the at least one return electrode.

7. The device of any preceding claim, wherein the first electrode (226) is supported on a body member configured for contact with an internal surface structure corresponding to and aligned with the macula of the eyeball.

8. The device of claim 1, wherein the second electrode (228) is supported on a body member (265, 270) configured for contact with an external surface structure (158, 159, 304) of the eyeball, in particular for corneal or epi-conjunctival contact with the external surface structure of the eyeball.

9. The device of any one of the preceding claims, wherein the first electrode (226) and the second electrode (228) are electrically interconnected via a wire.

## Patentansprüche

1. Vorrichtung zur Behandlung einer degenerativen Netzhauterkrankung, umfassend:
- eine Quelle (224, 224', 224") elektrischer Stimulation,
- eine an die Quelle (224, 224', 224") gekoppelte erste Elektrode (226), die für einen Kontakt mit einer ersten Innen-Oberflächenstruktur (300) eines Augapfels (220) posterior zur bulbären Konjunktiva und die verschiedenen Laminae, beginnend mit der Sklera als innerste Oberflächenstruktur und deren darüberliegende Strukturen, umfassend konfiguriert ist, und
- eine an die Quelle (224, 224', 224") gekoppelte zweite Elektrode (228), die für einen Kontakt mit einer zweiten Oberflächenstruktur des Augapfels (220) konfiguriert ist,
**dadurch gekennzeichnet, dass** die Quelle (224) eine innere Quellenkomponente (224') umfasst, die eine subkutan implantierte Empfänger-Induktionsspule aufweist, und weiter eine externe Quellenkomponente (224") umfasst, die eine Sende-Spule aufweist, die für die Übertragung von Energie und Daten konfiguriert ist, wobei die zweite Elektrode (228) für einen Kontakt mit einer Außen-Oberflächenstruktur (158, 159, 304) des Augapfels konfiguriert ist, oder dass die zweite Elektrode (228) für eine Anbringung an eine Innen-Oberflächenstruktur des Augapfels, die eine Außen-Oberfläche der Sklera ist, konfiguriert ist.

2. Vorrichtung nach Anspruch 1, wobei die zweite Elektrode (228) an einem Körperelement (281-283, 290) gehalten ist, welches für eine Anbringung an die Außen-Oberfläche der Sklera konfiguriert ist.

3. Vorrichtung nach Anspruch 2, wobei das Körperelement (281-283, 290) ein Band, einen Ring oder einen Mantel umfasst, welches bzw. welcher konfiguriert ist, sich zumindest teilweise um den Augapfel zu erstrecken, um die Positionierung und Fixierung der zweiten Elektrode zu unterstützen.

4. Vorrichtung nach Anspruch 3, wobei das Körperelement (281-283, 290) eine oder mehrere Öffnungen umfasst, um die vorhanden Augenstrukturen, wie zum Beispiel Verbindungen zwischen Muskeln und der Sklera, aufzunehmen.

5. Vorrichtung nach Anspruch 1, wobei die erste Elektrode (226) wenigstens eine Stimulationselektrode ist, die für einen chronischen Kontakt mit der ersten Oberflächenstruktur des Augapfels konfiguriert ist, und die zweite Elektrode (228) wenigstens eine Rückleitelektrode ist, die für einen chronischen Kontakt mit der zweiten Oberflächenstruktur des Augapfels konfiguriert ist.

6. Vorrichtung nach Anspruch 5, wobei dem Augapfel (220) ein elektrisches Stimulationssignal von der Quelle (224, 224', 224") über die wenigstens eine Stimulationselektrode und die wenigstens eine Rückleitelektrode zugeführt wird.

7. Vorrichtung nach einem der vorhergehenden Ansprüche, wobei die erste Elektrode (226) an einem Körperelement gehalten ist, welches für einen Kontakt mit einer Innen-Oberflächenstruktur, die einer Makula des Augapfels entspricht und an dieser ausgerichtet ist, konfiguriert ist.

8. Vorrichtung nach Anspruch 1, wobei die zweite Elektrode (228) an einem Körperelement (265, 270) gehalten ist, welches für einen Kontakt mit einer Außen-Oberflächenstruktur (158, 159, 304) des Augapfels, insbesondere für einen kornealen oder epi-konjunktivalen Kontakt mit der Außen-Oberflächenstruktur des Augapfels, konfiguriert ist.

9. Vorrichtung nach einem der vorhergehenden Ansprüche, wobei die erste Elektrode (226) und die zweite Elektrode (228) elektrisch über ein Kabel miteinander verbunden sind.

## Revendications

1. Dispositif de traitement de maladie rétinienne dégénérative comprenant :
une source (224, 224', 224") de stimulation électrique ;
une première électrode (226) couplée à la source (224, 224', 224"), configurée pour être en contact avec une première structure superficielle interne (300) d'un globe oculaire (220), postérieure à la conjonctive bulbaire et comprenant les différentes membranes commençant par la sclérotique en tant que structure superficielle la plus intérieure et ses structures sus-jacentes,
une seconde électrode (228) couplée à la source (224, 224', 224"), configurée pour être en contact avec une seconde structure superficielle du globe oculaire (220) ;
**caractérisé en ce que** la source (224) comprend un composant de source interne (224') présentant une bobine d'induction réceptrice implantée de manière sous-cutanée et comprend en outre un composant de source externe (224") présentant une bobine d'induction émettrice configurée pour transmettre de l'énergie et des données, dans lequel la seconde électrode (228) est configurée pour être en contact avec une structure superficielle externe (158, 159, 304) du globe oculaire, ou **en ce que** la seconde électrode (228) est configurée pour être fixée à une structure superficielle interne du globe oculaire correspondant à une surface externe de la sclère.

2. Dispositif selon la revendication 1, dans lequel la seconde électrode (228) est supportée sur un élément de corps (281-283, 290) configuré pour être fixé à la surface externe de la sclère.

3. Dispositif selon la revendication 2, dans lequel l'élément de corps (281-283, 290) comprend une bande, un anneau ou une gaine qui est configuré(e) pour s'étendre au moins partiellement autour du globe oculaire pour faciliter le positionnement et la fixation de la seconde électrode.

4. Dispositif selon la revendication 3, dans lequel l'élément de corps (281-283, 290) comprend une ou plusieurs ouvertures pour s'adapter à la présence de structures oculaires, telles que des liaisons entre des muscles et la sclère.

5. Dispositif selon la revendication 1, dans lequel la première électrode (226) est au moins une électrode de stimulation configurée pour être en contact chronique avec la première structure superficielle du globe oculaire, et la seconde électrode (228) est au moins une électrode de référence configurée pour être en contact chronique avec la seconde structure superficielle du globe oculaire.

6. Dispositif selon la revendication 5, dans lequel un signal de stimulation électrique provenant de la source (224, 224', 224") est appliqué au globe oculaire (220) par le biais de l'au moins une électrode de stimulation et de l'au moins une électrode de référence.

7. Dispositif selon l'une quelconque des revendications précédentes, dans lequel la première électrode (226) est supportée sur un élément de corps configuré pour être en contact avec une structure superficielle interne correspondant à et en alignement avec une macula du globe oculaire.

8. Dispositif selon la revendication 1, dans lequel la seconde électrode (228) est supportée sur un élément de corps (265, 270) configuré pour être en contact avec une structure superficielle externe (158, 159, 304) du globe oculaire, en particulier pour être en contact cornéen ou épi-conjonctival avec la structure superficielle externe du globe oculaire.

9. Dispositif selon l'une quelconque des revendications précédentes, dans lequel la première électrode (226) et la seconde électrode (228) sont interconnectées électriquement par le biais d'un fil.
